# FASCICULE DE BREVET EUROPEEN

(11) **EP 0 699 432 B1**
(45) Date de publication et mention de la délivrance du brevet: **15.01.1997**
(21) Numéro de dépôt: 95401516.0
(22) Date de dépôt: 26.06.1995
(51) Int. Cl.: A61K 7/48, A61K 7/06, A61K 35/08

(54) **Composition cosmétique et/ou dermatologique contenant de l'eau thermale ou minérale et un actif pour lutter contre l'acné ou le vieillissement**
Kosmetisches und/oder dermatologisches Mittel enthaltend Thermal- oder Mineralwasser und einen gegen Akne oder Älterung aktiven Agentsien
Cosmetic and/or dermatologic composition containing thermal or mineral water and an active agent against acne or ageing

(30) Priorité: 22.07.1994 FR 9409119
(43) Date de publication de la demande: 06.03.1996
(73) Titulaire: L'OREAL, 75008 Paris (FR)
(72) Inventeur: Koulbanis, Constantin, F-94270 Le Kremlin-Bicetre (FR); Laugier, Jean-Pierre, F-92160 Antony (FR); Gagnebien-Cabanne, Françoise, F-92320 Chatillon (FR); Deprez, Sabine, F-94320 Thiais (FR)
(74) Mandataire: Lhoste, Catherine

(56) Documents cités:
- WO-A-92/06666
- FR-A- 1 248 192
- FR-M- 3 574
- DATABASE WPI Week 9506 Derwent Publications Ltd., London, GB; AN 95-041193 & JP-A-06 321 789 (OGATA)
- DATABASE WPI Week 9509 Derwent Publications Ltd., London, GB; AN 95-065701 & SU-A-1 833 733 (BOGUCHAEVA ET AL.)
- DATABASE WPI Week 8026 Derwent Publications Ltd., London, GB; AN 80-45798C & JP-A-55 065 298 (EMPIRE BOEKI KK)

## Description

La présente invention se rapporte à une composition cosmétique et/ou dermatologique à base d'eau thermale et/ou minérale pour traiter en douceur la peau du visage et/ou du corps humain, y compris celle du cuir chevelu, tout en l'hydratant. Elle se rapporte aussi à l'utilisation de cette composition et à un procédé pour le traitement cosmétique de l'acné, des rides et/ou des ridules de la peau.

Les actifs couramment utilisés pour ces traitements sont des agents kératolytiques choisis parmi les hydroxyacides α et β et leurs dérivés, les rétinoïdes et leurs dérivés et notamment l'acide rétinoïque et le rétinol (tout trans, 13-cis), le peroxyde de benzoyle.

Parmi les β-hydroxyacides, on trouve notamment l'acide salicylique et ses dérivés. L'acide salicylique est connu pour le traitement de l'acné (voir EP-A-281812) et des rides (voir le document WO-A 93/10755), et certains des dérivés de cet acide sont connus pour le traitement du vieillissement cutané et notamment pour retarder et/ou atténuer l'apparition des rides et ridules (voir EP-A-378 936).

L'acide rétinoïque est également connu pour prévenir l'apparition des rides et/ou ridules sur la peau.

Par ailleurs, ces agents kératolytiques sont connus pour modifier le teint de la peau qui apparaît plus rose, pour faire disparaître les taches pigmentées présentes en surface, pour supprimer les squames ainsi qu'améliorer l'élasticité de la peau.

Malheureusement, une fois appliqués sur la peau, ces agents kératolytiques entraînent des démangeaisons, des picotements, des tiraillements pouvant conduire à d'importants inconforts, du fait de l'agression qu'ils provoquent sur la kératine de la peau.

De plus, l'utilisation de ces produits par des consommateurs à peau sensible est souvent rédhibitoire.

L'eau minérale présente sous forme de liposomes dans des compositions cosmétiques est déjà connue, par le document FR-A-2 608 426, en vue d'hydrater les couches superficielles et profondes de la peau.

Par ailleurs, le document FR-2-668 063 enseigne l'utilisation de liposomes d'eau thermale stabilisés dans un gel d'ADN pour la préparation de compositions cosmétiques. Une telle forme d'utilisation de l'eau thermale permet, selon ce document, sa pénétration ciblée dans le derme et l'épiderme de la peau.

Malheureusement, ces compositions liposomées à base d'eau thermale ou d'eau minérale ne permettent pas le traitement des rides, des ridules et/ou de l'acné présents sur la peau du visage et/ou du corps humain ou sur le cuir chevelu.

Aussi, il subsiste le besoin d'une composition cosmétique et/ou dermatologique à base d'eau thermale et/ou minérale qui permette à la peau d'être rajeunie et qui permette d'éliminer les boutons d'acné, tout en évitant les inconvénients mentionnés précédemment et notamment en favorisant l'hydratation de la peau en surface et en profondeur.

La demanderesse a constaté que les agents kératolytiques mentionnés précédemment agissaient notamment par un mécanisme d'inflammation de la peau, légère mais, suffisante pour provoquer après quelques mois d'application sur celle-ci cet aspect de rajeunissement et de bonne mine.

Ainsi, afin de conserver les propriétés inflammatoires de ces agents kératolytiques tout en permettant l'hydratation de la peau, la demanderesse a envisagé d'utiliser une eau thermale ou minérale ayant une minéralisation d'au moins 400 mg/l dans une composition cosmétique et/ou dermatologique, contenant un ou plusieurs agents kératolytiques.

Ainsi, l'invention se rapporte à l'utilisation d'une eau ayant une minéralisation d'au moins 400 mg/l, dans une composition cosmétique et/ou dermatologique contenant au moins un actif ayant un effet secondaire irritant, pour éliminer cet effet irritant. L'eau utilisée est notamment une eau thermale et/ou minérale.

L'invention se rapporte également à une composition cosmétique et/ou dermatologique comprenant au moins un actif ayant un effet secondaire irritant, dans un milieu cosmétiquement et/ou dermatologiquement acceptable, et comprenant un agent apaisant choisi parmi les eaux thermales et/ou les eaux minérales ayant une minéralisation d'au moins 700 mg/l et une concentration totale en ions carbonates et bicarbonates d'au moins 360 mg/l.

On connaît du document JP-A-55-65298 une composition détergente aqueuse, appropriée notamment pour laver la vaisselle, obtenue par dissolution d'un sel d'acylaminoacide et d'un ester gras de sucrose dans une eau minérale dont la teneur en carbonates est de 110 à 170 mg/l.

On entend, dans l'invention, par "minéralisation", la somme des concentrations en anions et en cations présents dans l'eau thermale ou minérale. Le fait d'utiliser une eau de forte minéralisation permet justement de compenser l'effet irritant des actifs cosmétiques et/ou dermatologiques comme les kératolytiques.

La présente invention peut utiliser indifféremment une eau thermale ou une eau minérale. En général, une eau minérale est propre à la consommation, ce qui n'est pas toujours le cas d'une eau thermale. Chacune de ces eaux contient, entre autre, des minéraux solubilisés et des oligoéléments. Ces eaux sont connues pour être employées à des fins de traitement spécifique selon les oligoéléments et les minéraux particuliers qu'elles contiennent, tels que l'hydratation et la désensibilisation de la peau ou le traitement de certaines dermatoses. Aussi, selon le type d'eau utilisée, on peut, en plus de l'apaisement de l'effet secondaire irritant de certains actifs, traiter spécifiquement la peau.

En effet, plus une eau est minéralisée, plus elle est difficile à incorporer dans des compositions du fait de la formation aisée de précipités lors de la préparation de ces compositions.

L'eau thermale et/ou minérale utilisée selon l'invention peut avoir une minéralisation d'au moins 700 mg/l et, en particulier, une concentration totale en carbonates et en bicarbonates d'au moins 150 mg/l et plus préférentiellement d'au moins 360 mg/l et notamment en carbonate et bicarbonate de sodium supérieure à 2 mg/l. La concentration en oxyde de silicium dans l'eau utilisée dans la composition selon l'invention peut être de préférence d'au moins 6 mg/l et plus préférentiellement d'au moins 9 mg/l.

L'eau thermale ou l'eau minérale utilisée selon l'invention peut être choisie parmi l'eau de Vittel, les eaux du bassin de Vichy, l'eau d'Uriage, l'eau de la Roche Posay, l'eau de la Bourboule, l'eau d'Enghien-les-bains, l'eau de Saint-Gervais-les bains, l'eau de Néris-les-bains, l'eau d'Allevard-les-bains, l'eau de Digne, l'eau des Maizières, l'eau de Neyrac-les-bains, l'eau de Lons le Saunier, les Eaux Bonnes, l'eau de Rochefort, l'eau de Saint Christau, l'eau des Fumades et l'eau de Tercis-les-bains.

Parmi ces eaux, celles qui présentent une minéralisation inférieure à 700 mg/l mais supérieure 400 mg/l sont l'eau de la Roche Posay, les Eaux Bonnes, l'eau de Saint Christau.

Parmi ces eaux, celles qui présentent une concentration totale en carbonates ou bicarbonates supérieure à 360 mg/l sont l'eau de Vittel, l'eau de la Bourboule, l'eau des Fumades, l'eau d'Enghien-les-bains, l'eau de la Roche-Posay, l'eau du bassin de Vichy, l'eau d'Uriage.

Parmi ces eaux celles qui présentent une concentration en carbonates ou bicarbonates comprise entre 150 mg/l et 360 mg/l sont l'eau de Digne, l'eau de Maizières, l'eau de Rochefort, l'eau de Saint-Gervais-les-bains.

Parmi ces eaux, celles qui contiennent au moins 2 mg/l de carbonate ou bicarbonate de sodium sont l'eau de la Roche Posay, l'eau de Vittel, les eaux du bassin de Vichy, l'eau d'Uriage

Les eaux contenant au moins 9 mg/l d'oxyde de silicium sont l'eau de la Roche Posay, l'eau de Vittel, les eaux du bassin de Vichy, l'eau d'Uriage.

L'agent à effet secondaire irritant est, en particulier, un agent kératolytique mais pourrait être aussi un antifongique, antibactérien, antiséborrhéïque.

L'agent kératolytique, utilisé selon l'invention, peut être choisi parmi les α-hydroxyacides (acide glycolique, mandélique, lactique, tatrique, citrique), les β-hydroxyacides et leurs dérivés, les rétinoïdes (acide rétinoïque ou rétinol) et leurs dérivés, le peroxyde de benzoyle, ainsi que les sels de ces différents composés.

L'agent kératolytique est de préférence un β-hydroxyacide, comme l'acide salicylique ou un de ses dérivés, éventuellement salifié. Préférentiellement, l'agent kératolytique est un dérivé d'acide salicylique éventuellement salifié. Comme dérivés de l'acide salicylique, on peut citer notamment ceux décrits dans les documents FR-A-2581542 et EP-A-378936, et notamment les acides n-octanoyl-5-salicylique, n-décanoyl-5-salicylique, n-dodécanoyl-5-salicylique, n-octyl-5-salicylique, n-heptyloxy-5-salicylique, n-heptyloxy-4-salicylique, ainsi que leurs sels d'ammonium quaternaire tels que les sels de diméthylhydroxypropylammonium. On peut aussi utiliser les dérivés d'acide salicylique décrits dans le document EP-A-570230.

De façon préférée, la composition selon l'invention comprend de l'eau du bassin de Vichy et de l'acide n-octanoyl-5-salicylique.

La proportion en agent kératolytique dans la composition selon l'invention va, par exemple, de 0,1 % à 5 % en poids par rapport au poids total de la composition. Cette proportion va de préférence de 0,1 % à 2 % en poids par rapport au poids total de la composition.

L'eau thermale ou minérale est choisie de préférence en une quantité supérieure à 0,1 % en poids par rapport au poids total de la composition et par exemple au moins égale à 5 % en poids, et mieux allant de 5 % à 75 %. L'eau thermale ou minérale peut être additionnée d'eau distillée ou déminéralisée dans la composition.

Un autre avantage de la composition selon l'invention est de pouvoir utiliser, dans des compositions cosmétiques et/ou dermatologiques, des eaux thermales ou minérales ayant à la fois des concentrations élevées en ions carbonates et bicarbonates, et une concentration élevée en ion positif, tel que le sodium et le calcium, sans craindre la formation d'un précipité calcique, du fait de la nature acide de l'agent kératolytique.

La composition selon l'invention peut se présenter sous forme d'une solution, d'un gel aqueux, d'une émulsion (huile-dans-eau ou eau-dans-huile) ou d'une dispersion de vésicules lipidiques et avoir l'aspect d'une lotion, d'un sérum, d'une crème ou d'un gel. Elle peut comprendre, en outre, au moins un actif lipophile ou hydrophile différent de l'agent à effet secondaire irritant et notamment choisi parmi les filtres, les céramides, les protéines et leurs hydrolysats, les hydratants.

La composition selon l'invention peut, en outre, comprendre au moins un additif cosmétiquement et/ou dermatologiquement acceptable choisi parmi les tensioactifs (émulsionnants et coémulsionnants), les corps gras, des conservateurs, des parfums, des gélifiants, des complexants, des neutralisants.

La présente invention se rapporte aussi à l'utilisation de la composition définie ci-dessus pour le traitement cosmétique de l'acné, des rides, et/ou des ridules de la peau et à l'utilisation de cette composition pour la préparation d'une pommade dermatologique destinée au traitement de l'acné, des rides et/ou des ridules. Elle se rapporte encore à un procédé de traitement cosmétique de l'acné, des rides et/ou des ridules de la peau, consistant à appliquer sur la peau la composition définie précédemment.

On donne ci-après un exemple de composition conforme à l'invention. Les quantités y sont données en pourcentage pondéral.

### Exemple : Emulsion H/E destinée au traitement de la peau du visage

### Phase grasse :

| | |
|---|---|
| - Huile d'amandes d'abricot (triglycérides d'acides oléique-linoléïque) | 14,5 % |
| - Fraction liquide de beurre de karité (Triglycérides d'acide palmitique-stéarique-oléïque-linoléïque) | 7,0 % |
| - Parahydroxybenzoate de propyle (conservateur) | 0,1 % |
| - Mélange d'alcool gras (Alcool stéarylique, alcool arachidylique, alcool béhénylique) | 1,0 % |
| - Mono-stéarate de sorbitane (Span 60 de chez ICI) | 2,5 % |
| - Mélange d'éthyl-2 héxanoate de cétylstéaryle, myristate d'isopropyle (huile de purcellin) | 2,0 % |

### Phase aqueuse :

| | |
|---|---|
| - Conservateurs | 0,5 % |
| - Sel disodique de l'acide éthylène di-amine tétracétique 2H₂O (complexants) | 0,05 % |
| - Neutralisant | 0,5 % |
| - Gélifiant | 0,7 % |
| - Glycérine | 5,0 % |
| - Monostéarate de sorbitane oxyéthyléné (20OE) (Tween 60 de ICI) (tensioactif) | 2,5 % |
| - Acide n-octanyol-5-salicylique | 1 % |
| - Eaux du bassin de Vichy | 62,65 % |
| - Eau déminéralisée ou permutée | qsp 100 % |

L'émulsion se présente sous forme d'une crème blanche destinée au traitement des rides, dues au vieillissement, et à l'hydratation de la peau.

Parmi les différentes eaux minérales et/ou thermales mentionnées ci-dessus, la demanderesse a trouvé, grâce à l'aide de tests comparatifs, que L'eau du bassin de Vichy avait un pouvoir d'apaisement de l'irritation due à un agent kératolytique supérieur à celui des autres eaux.

Ainsi, on a mesuré l'activité anti-irritante de compositions cosmétiques contenant 2 % d'acide n-octanoyl-5-salicylique dans les eaux du bassin de Vichy, sur l'homme.

On a obtenu les résultats suivants :

| **Eau thermale** | **% d'inhibition de l'effet irritant** |
|---|---|
| - Eau du bassin de Vichy | 51 |
| - Eau de Vittel | 24 |
| - Eau de La Roche Posay | 46 |

## Revendications

1. Utilisation d'une eau ayant une minéralisation d'au moins 400 mg/l dans une composition cosmétique et/ou dermatologique contenant au moins un actif ayant un effet secondaire irritant, pour éliminer cet effet irritant.

2. Utilisation selon la revendication 1, caractérisée en ce que l'eau est une eau thermale et/ou minérale.

3. Utilisation selon la revendication 2, caractérisée en ce que l'eau thermale et/ou minérale a une minéralisation supérieure à 700 mg/l.

4. Utilisation selon la revendication 2 ou 3, caractérisée en ce que l'eau thermale et/ou minérale a une concentration totale en ions carbonates et bicarbonates d'au moins 150 mg/l.

5. Utilisation selon l'une quelconque des revendications 2 à 4, caractérisée en ce que l'eau thermale a une concentration en oxyde de silicium d'au moins 6 mg/l.

6. Utilisation selon l'une quelconque des revendications précédentes, caractérisée en ce que l'eau est une eau thermale choisie parmi l'eau de Vittel, les eaux du bassin de Vichy, l'eau d'Uriage, l'eau de la Roche-Posay, l'eau de la Bourboule, l'eau des Fumades, l'eau d'Enghien-les-bains, les Eaux Bonnes.

7. Utilisation selon l'une des revendications 1 à 6, caractérisée en ce que l'actif ayant un effet secondaire irritant est un agent kératolytique.

8. Utilisation selon l'une quelconque des revendications précédentes, caractérisée en ce que l'agent kératolytique est choisi parmi l'acide salicylique et ses dérivés, les α-hydroxyacides et leurs dérivés, les rétinoïdes et leurs dérivés, le peroxyde de benzoyle ainsi que leurs sels.

9. Utilisation selon l'une quelconque des revendications précédentes, caractérisée en ce que l'actif est un dérivé d'acide salicylique.

10. Utilisation selon l'une quelconque des revendications précédentes, caractérisée en ce que l'actif est choisi parmi l'acide n-octanoyl-5-salicylique, l'acide n-dodécanoyl-5-salicylique, l'acide n-décanoyl-5-salicylique, ainsi que leurs sels d'ammonium quaternaire.

11. Composition cosmétique et/ou dermatologique comprenant au moins un actif ayant un effet secondaire irritant, dans un milieu cosmétiquement et/ou dermatologiquement acceptable, caractérisée en ce qu'elle comprend, en outre, au moins un agent apaisant choisi parmi les eaux thermales et/ou les eaux minérales ayant une minéralisation d'au moins 700 mg/l et une concentration totale en ions carbonates et bicarbonates d'au moins 360 mg/l.

12. Composition selon la revendication 11, caractérisée en ce que la concentration totale en carbonates et bicarbonates de sodium est supérieure à 2 mg/l.

13. Composition selon l'une quelconque des revendications 11 et 12, caractérisée en ce que l'eau thermale est choisie parmi l'eau de Vittel, l'eau du bassin de Vichy, l'eau d'Uriage, l'eau de la Bourboule, l'eau des Fumades, l'eau d'Enghien-les-bains.

14. Composition selon l'une quelconque des revendications 11 à 13, caractérisée en ce que l'actif ayant un effet secondaire irritant est un agent kératolytique.

15. Composition selon l'une quelconque des revendications 11 à 14, caractérisée en ce que l'agent kératolytique est choisi parmi l'acide salicylique et ses dérivés, les α-hydroxyacides et leurs dérivés, les rétinoïdes et leurs dérivés, le peroxyde de benzoyle ainsi que leurs sels.

16. Composition selon l'une quelconque des revendications 11 à 15, caractérisée en ce que l'agent kératolytique est choisi parmi l'acide n-octanoyl-5-salicylique, l'acide n-dodécanoyl-5-salicylique, l'acide n-décanoyl-5-salicylique et leurs sels d'ammonium quaternaire.

17. Composition selon l'une quelconque des revendications 11 à 16, caractérisée en ce que la concentration en actif va de 0,1 % à 5 % en poids par rapport au poids total de la composition.

18. Composition selon l'une quelconque des revendications 11 à 17, caractérisée en ce que la concentration en actif va de préférence de 0,1 % à 2 % en poids par rapport au poids total de la composition.

19. Composition selon l'une quelconque des revendications 11 à 18, caractérisée en ce que la concentration en eau thermale est au moins supérieure à 5 % en poids par rapport au poids total de la composition.

20. Composition selon l'une quelconque des revendications 11 à 19, caractérisée en ce qu'elle est sous forme d'une solution, d'un gel aqueux, d'une émulsion ou d'une dispersion de vésicules lipidiques.

21. Composition selon l'une quelconque des revendications 11 à 20, caractérisée en ce qu'elle comprend, en outre, des actifs lipophiles ou hydrophiles différents d'un agent kératolytique.

22. Utilisation de la composition selon l'une des revendications précédentes pour le traitement cosmétique de l'acné, des rides et/ou des ridules.

23. Procédé de traitement cosmétique de l'acné, des rides et/ou des ridules de la peau, caractérisé en ce qu'il consiste à appliquer sur la peau la composition selon l'une des revendications 11 à 21.

## Claims

1. Use of a water having a mineralization of at least 400 mg/l in a cosmetic and/or dermatological composition containing at least one active agent having an irritant side effect, in order to remove this irritant effect.

2. Use according to Claim 1, characterized in that the water is a thermal spring water and/or mineral water.

3. Use according to Claim 2, characterized in that the thermal spring water and/or mineral water has a mineralization of greater than 700 mg/l.

4. Use according to Claim 2 or 3, characterized in that the thermal spring water and/or mineral water has a total concentration of carbonate and bicarbonate ions of at least 150 mg/l.

5. Use according to any one of Claims 2 to 4, characterized in that the thermal spring water has a concentration of silicon oxide of at least 6 mg/l.

6. Use according to any one of the preceding claims, characterized in that the water is a thermal spring water chosen from eau de Vittel, eaux du bassin de Vichy, eau d'Uriage, eau de la Roche Posay, eau de la Bourboule, eau des Fumades, eau d'Enghien-les-bains and Eaux Bonnes.

7. Use according to one of Claims 1 to 6, characterized in that the active agent having an irritant side effect is a keratolytic agent.

8. Use according to any one of the preceding claims, characterized in that the keratolytic agent is chosen from salicylic acid and derivatives thereof, α-hydroxy acids and derivatives thereof, retinoids and derivatives thereof, benzoyl peroxide, and the salts thereof.

9. Use according to any one of the preceding claims, characterized in that the active agent is a salicylic acid derivative.

10. Use according to any one of the preceding claims, characterized in that the active agent is chosen from 5-n-octanoylsalicylic acid, 5-n-dodecanoylsalicylic acid, 5-n-decanoylsalicylic acid, and the quaternary ammonium salts thereof.

11. Cosmetic and/or dermatological composition comprising at least one active agent having an irritant side effect, in a cosmetically and/or dermatologically acceptable medium, characterized in that it additionally comprises at least one calming agent chosen from thermal spring waters and/or mineral waters having a mineralization of at least 700 mg/l and a total concentration of carbonate and bicarbonate ions of at least 360 mg/l.

12. Composition according to Claim 11, characterized in that the total concentration of sodium carbonates and bicarbonates is greater than 2 mg/l.

13. Composition according to either of Claims 11 and 12, characterized in that the thermal spring water is chosen from eau de Vittel, eau du bassin de Vichy, eau d'Uriage, eau de la Bourboule, eau des Fumades and eau d'Enghien-les-bains.

14. Composition according to any one of Claims 11 to 13, characterized in that the active agent having an irritant side effect is a keratolytic agent.

15. Composition according to any one of Claims 11 to 14, characterized in that the keratolytic agent is chosen from salicylic acid and derivatives thereof, α-hydroxy acids and derivatives thereof, retinoids and derivatives thereof, benzoyl peroxide, and salts thereof.

16. Composition according to any one of Claims 11 to 15, characterized in that the keratolytic agent is chosen from 5-n-octanoylsalicylic acid, 5-n-dodecanoylsalicylic acid, 5-n-decanoylsalicylic acid, and the quaternary ammonium salts thereof.

17. Composition according to any one of Claims 11 to 16, characterized in that the concentration of active agent ranges from 0.1 % to 5 % by weight relative to the total weight of the composition.

18. Composition according to any one of Claims 11 to 17, characterized in that the concentration of active agent preferably ranges from 0.1 % to 2 % by weight relative to the total weight of the composition.

19. Composition according to any one of Claims 11 to 18, characterized in that the concentration of thermal spring water is at least greater than 5 % by weight relative to the total weight of the composition.

20. Composition according to any one of Claims 11 to 19, characterized in that it is in the form of a solution, an aqueous gel, an emulsion or a dispersion of lipid vesicles.

21. Composition according to any one of Claims 11 to 20, characterized in that it additionally comprises lipophilic or hydrophilic active agents other than a keratolytic agent.

22. Use of the composition according to one of the preceding claims for the cosmetic treatment of acne, wrinkles and/or fine lines.

23. Process for the cosmetic treatment of acne, wrinkles and/or fine lines on the skin, characterized in that it consists in applying the composition according to one of Claims 11 to 21 to the skin.

## Patentansprüche

1. Verwendung eines Wassers mit einem Mineralstoffgehalt von mindestens 400 mg/l in einer kosmetischen und/oder dermatologischen Zusammensetzung, die mindestens einen Wirkstoff enthält, der eine Reizwirkung als Nebenwirkung aufweist, zur Beseitigung dieser Reizwirkung.

2. Verwendung nach Anspruch 1, dadurch gekennzeichnet, daß das Wasser ein Thermalwasser und/oder ein Mineralwasser ist.

3. Verwendung nach Anspruch 2, dadurch gekennzeichnet, daß das Thermalwasser und/oder Mineralwasser einen Mineralstoffgehalt von über 700 mg/l aufweist.

4. Verwendung nach Anspruch 2 oder 3, dadurch gekennzeichnet, daß das Thermalwasser und/oder Mineralwasser eine Gesamtkonzentration an Carbonationen und Hydrogencarbonationen von mindestens 150 mg/l aufweist.

5. Verwendung nach einem der Ansprüche 2 bis 4, dadurch gekennzeichnet, daß das Thermalwasser eine Konzentration an Siliciumdioxid von mindestens 6 mg/l aufweist.

6. Verwendung nach einem der vorhergehenden Ansprüche, dadurch gekennzeichnet, daß das Wasser ein Thermalwasser ist, das unter Wasser aus Vittel, dem Vichy-Becken, Uriage, la Roche-Posay, Bourboule, Fumades, Enghien-les-bains und Eaux Bonnes ausgewählt ist.

7. Verwendung nach einem der Ansprüche 1 bis 6, dadurch gekennzeichnet, daß der Wirkstoff, der eine Reizwirkung als Nebenwirkung aufweist, ein Keratolytikum ist.

8. Verwendung nach einem der vorhergehenden Ansprüche, dadurch gekennzeichnet, daß das Keratolytikum unter Salicylsäure und ihren Derivaten, α-Hydroxysäuren und ihren Derivaten, Retinoidverbindungen und ihren Derivaten, Benzoylperoxid sowie deren Salzen ausgewählt ist.

9. Verwendung nach einem der vorhergehenden Ansprüche, dadurch gekennzeichnet, daß der Wirkstoff ein Salicylsäurederivat ist.

10. Verwendung nach einem der vorhergehenden Ansprüche, dadurch gekennzeichnet, daß der Wirkstoff unter 5-n-Octanoylsalicylsäure, 5-n-Dodecanoylsalicylsäure und 5-n-Decanoylsalicylsäure sowie ihren quaternären Ammoniumsalzen ausgewählt ist.

11. Kosmetische und/oder dermatologische Zusammensetzung, die mindestens einen Wirkstoff mit einer Reizwirkung als Nebenwirkung in einem kosmetischen und/oder dermatologisch akzeptablen Medium enthält, dadurch gekennzeichnet, daß sie ferner mindestens ein linderndes Mittel enthält, das ausgewählt ist unter den Thermalwässern und/oder Mineralwässern mit einem Mineralstoffgehalt von mindestens 700 mg/l und einer Gesamtkonzentration an Carbonationen und Hydrogencarbonationen von mindestens 360 mg/l.

12. Zusammensetzung nach Anspruch 11, dadurch gekennzeichnet, daß die Gesamtkonzentration an Natriumcarbonat und Natriumhydrogencarbonat über 2 mg/l liegt.

13. Zusammensetzung nach Anspruch 11 oder 12, dadurch gekennzeichnet, daß das Thermalwasser ausgewählt ist unter Wasser aus Vittel, Wasser aus dem Vichy-Becken, Wasser aus Uriage, Wasser aus Bourboule, Wasser aus Fumades und Wasser aus Enghien-les-bains.

14. Zusammensetzung nach einem der Ansprüche 11 bis 13, dadurch gekennzeichnet, daß das Mittel mit einer Reizwirkung als Nebenwirkung ein Keratolytikum ist.

15. Zusammensetzung nach einem der Ansprüche 11 bis 14, dadurch gekennzeichnet, daß das Keratolytikum unter Salicylsäure und ihren Derviaten, α-Hydroxysäuren und ihren Derivaten, Retinoidverbindungen und ihren Derivaten und Benzoylperoxid sowie deren Salzen ausgewählt ist.

16. Zusammensetzung nach einem der Ansprüche 1 bis 15, dadurch gekennzeichnet, daß das Keratolytikum unter 5-n-Octanoylsalicylsäure, 5-n-Dodecanoylsalicylsäure und 5-n-Decanoylsalicylsäure sowie deren quaternären Ammoniumsalzen ausgewählt ist.

17. Zusammensetzung nach einem der Ansprüche 11 bis 16, dadurch gekennzeichnet, daß die Wirkstoffkonzentration im Bereich von 0,1 bis 5 Gew.-%, bezogen auf das Gesamtgewicht der Zusammensetzung, liegt.

18. Zusammensetzung nach einem der Ansprüche 11 bis 17, dadurch gekennzeichnet, daß die Wirkstoffkonzentration vorzugsweise im Bereich von 0,1 bis 2 Gew.-%, bezogen auf das Gesamtgewicht der Zusammensetzung, liegt.

19. Zusammensetzung nach einem der Ansprüche 11 bis 18, dadurch gekennzeichnet, daß die Konzentration an Thermalwasser mindestens über 5 Gew.-%, bezogen auf das Gesamtgewicht der Zusammenseztung, beträgt.

20. Zusammensetzung nach einem der Ansprüche 11 bis 19, dadurch gekennzeichnet, daß sie in Form einer Lösung, als wässeriges Gel, als Emulsion oder als Dispersion von Lipidvesikeln vorliegt.

21. Zusammensetzung nach einem der Ansprüche 11 bis 20, dadurch gekennzeichnet, daß sie ferner lipophile oder hydrophile Wirkstoffe enthält, die keine Keratolytika sind.

22. Verwendung der Zusammensetzung nach einem vorhergehenden Ansprüche zur kosmetischen Behandlung von Akne, Falten und/oder Fältchen.

23. Verfahren zur kosmetischen Behandlung von Akne, Falten und/oder Fältchen der Haut, dadurch gekennzeichnet, daß es darin besteht, die Zusammensetzung nach einem der Ansprüche 11 bis 21 auf die Haut aufzutragen.
